(19) Europäisches Patentamt
European Patent Office
Office européen des brevets

(11) Publication number: **0 140 571**
**A1**

# (12) EUROPEAN PATENT APPLICATION

(21) Application number: **84306453.6**

(22) Date of filing: **21.09.84**

(51) Int. Cl.⁴: **C 07 D 249/08**, C 07 D 233/60, A 01 N 43/653, A 01 N 43/50

(30) Priority: **30.09.83 GB 8326210**

(43) Date of publication of application: **08.05.85 Bulletin 85/19**

(84) Designated Contracting States: **AT BE CH DE FR GB IT LI LU NL SE**

(71) Applicant: **FBC LIMITED, Hauxton, Cambridge CB2 5HU (GB)**

(72) Inventor: **Gates, Peter Stuart, 187 Campkin Road, Cambridge CB4 2LE (GB)**

(74) Representative: **Waldman, Ralph David et al, Industrial Property Department FBC Limited Chesterford Park Research Station, Saffron Walden, Essex CB10 1XL (GB)**

## (54) Diazole and triazole compounds.

(57) Compounds of formula I

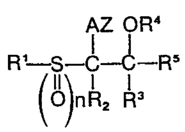

I

where

i) Az is 1-imidazolyl or 1-(1,2,4-triazolyl);

ii) $R^1$ and $R^5$ are aryl, cycloalkyl or optionally substituted alkyl;

iii) $R^4$ is hydrogen, alkenyl, cycloalkyl, optionally substituted alkyl, aryl, optionally substituted alkanoyl, optionally substituted benzoyl, alkoxycarbonyl or optionally substituted carbamoyl;

iv) n is 0,1 or 2;

v) (a) $R^2$ is hydrogen, optionally substituted alkyl, cycloalkyl or alkenyl and $R^3$ is hydrogen or optionally substituted alkyl, or

(b) $R^2$ and $R^3$ form a double bond;

vi) when $R^2$ is optionally substituted alkyl, cycloalkyl or alkenyl and Az is 1-(1,2,4-triazolyl), $R^3$ and $R^4$ can also form a double bond;

together with acid addition salts, complexes of these compounds with metal salts, with the provisos

a) at least one of $R^2$, $R^3$ and $R^4$ is not hydrogen,

b) when $R^1$ is aryl and $R^2$ and $R^3$ are hydrogen, $R^4$ is not alkoxymethyl,

c) when $R^1$ is aryl or aralkyl, $R^2$ and $R^3$ are hydrogen and $R^5$ is unsubstituted alkyl, $R^4$ is not alkanoyl, and

d) when $R^2$ is substituted benzyl and $R^3$ and $R^4$ form a double bond, $R^5$ is not tertiary butyl, have fungicidal and plant growth regulant activity.

EP 0 140 571 A1

Case 83/26210

The present invention relates to compounds having use in agriculture.

There are many publications disclosing triazole and imidazole derivatives as fungicides and plant growth regulators. Relatively few however disclose compounds in which a substituted thio group is attached to the carbon carrying the azole group. Publications describing compounds of this type include G.B. Patent 1419734, EP 752, EP 89703, Japanese Kokai 57150675, 57149279 and 55124769.

The present invention relates to a novel group of azole derivatives of this type having valuable biological activity.

Thus according to the invention there is provided a compound of formula I

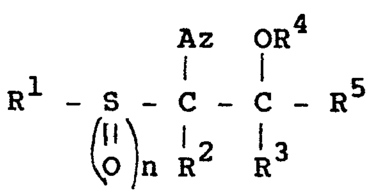

I

where i) Az is 1-imidazolyl or 1-(1,2,4-triazolyl);

ii) $R^1$ and $R^5$ are aryl, cycloalkyl or optionally substituted alkyl;

iii) $R^4$ is hydrogen, alkenyl, cycloalkyl,

optionally substituted alkyl, aryl, optionally substituted alkanoyl, optionally substituted benzoyl, alkoxycarbonyl or optionally substituted carbamoyl;

iv) n is 0,1 or 2;

v) (a) $R^2$ is hydrogen, optionally substituted alkyl, cycloalkyl or alkenyl and $R^3$ is hydrogen or optionally substituted alkyl, or

(b) $R^2$ and $R^3$ form a double bond;

vi) when $R^2$ is optionally substituted alkyl, cycloalkyl or alkenyl and Az is 1-(1,2,4-triazolyl), $R^3$ and $R^4$ can also form a double bond;

together with acid addition salts, complexes of these compounds with metal salts, with the provisos

a) at least one of $R^2$, $R^3$ and $R^4$ is not hydrogen,

b) when $R^1$ is aryl and $R^2$ and $R^3$ are hydrogen, $R^4$ is not alkoxymethyl,

c) when $R^1$ is aryl or aralkyl, $R^2$ and $R^3$ are hydrogen and $R^5$ is unsubstituted alkyl, $R^4$ is not alkanoyl, and

d) when $R^2$ is substituted benzyl and $R^3$ and $R^4$ form a double bond, $R^5$ is not tertiary butyl.

The complexes are usually with a salt of formula $MA_2$ in which M is a divalent metal cation, e.g. copper, calcium, cobalt, nickel or preferably manganese, and A is an anion, e.g. chloride, nitrate or a hydrocarbon sulphonate e.g. dodecylbenzenesulphonate. The molar ratio of the compound to metal salt is usually 2 or 4 to 1. Acid addition salts are usually formed with strong inorganic or organic acids e.g. hydrochloric acid or oxalic acid. Generally however the compounds are used as the free base.

Aryl groups are usually optionally substituted phenyl but may also include other aromatic groups such as naphthyl and heterocyclic aromatic groups which may be substituted, such as pyridyl, pyrimidyl, triazolyl, imidazolyl, thienyl and furanyl. When the aryl is substituted, the substituents may include halogen, hydroxy alkyl, alkoxy, cyano, nitro, aryl, or aryloxy. Preferred substituents are halogen, especially chlorine. Any alkyl and alkoxy groups are generally of 1 to 8, e.g. 1 to 4, carbon atoms and may be substituted e.g. by halogen, especially fluorine or chlorine, alkoxy, alkoxycarbonyl, 1,3-dioxolan-2-yl, alkanoyloxy or aryl. Alkenyl groups are

generally of 2 to 4 carbon atoms. Cycloalkyl groups are usually of 3 to 7 carbon atoms.

Az is preferably 1-(1,2,4-triazolyl) and n is preferably 0. Of the other groups, $R^1$ is preferably $C_{3-5}$ alkyl, especially tertiary butyl, or halophenyl, especially 4-chloro- or 2,4-dichlorophenyl; $R^2$, when not forming a double bond, is preferably hydrogen, methyl or ethyl; $R^3$, when not forming a double bond, is preferably hydrogen or methyl; $R^4$ is preferably alkyl, e.g. $C_{1-4}$ alkyl, optionally substituted by phenoxy, halophenyl, 1,3-dioxolan-2-yl, halophenyl, hydroxy, alkoxycarbonyl or alkanoyloxy; and $R^5$ is preferably alkyl, e.g. $C_{1-4}$ alkyl, or phenyl, substituted by halogen, (e.g. 4-chloro-, 2,4-dichloro- or 4-bromophenyl), haloalkoxy, e.g. difluoromethoxy, or trifluoromethyl.

A particularly preferred group of compounds is that where Az is 1-(1,2,4-triazolyl), n is O, $R^1$ is $C_{4-5}$ alkyl or halophenyl, $R^2$ and $R^3$ are hydrogen or methyl or together form a double bond, $R^4$ is $C_{1-4}$ alkyl and $R^5$ is $C_{1-4}$ alkyl or halophenyl.

The compounds of the invention have activity as fungicides, especially against fungal diseases of plants, e.g. mildews, such as powdery mildews and particularly barley powdery mildew (Erysiphe graminis), rice blast (Pyricularia oryzae), wheat brown rust (Puccinia

recondita), potato blight (Phytophthora infestans) and vine downy mildew (Plasmopora viticola).

The invention thus also provides a method of combating fungi at a locus infested or liable to be infested therewith, which comprises applying to the locus a compound of formula I.

Some of the compounds of the invention are effective in controlling the growth of a wide variety of crops, especially to cause height reduction without any detrimental effect on the health and vigour of the plants. The invention thus also includes a method of controlling plant growth by applying to the plant a growth regulating amount of a compound of formula I. This aspect of the invention is applicable to both mono- and dicotyledonous plants, e.g. mung beans, soybeans, barley, wheat, rice, sugarbeet, cotton, sunflowers, pot plants, such as chrysanthemums, turf grass, top fruit (e.g. apples), vegetables and woody ornamentals. In some cases,the products can encourage the formation of extra shoots or tillers e.g. on cereals.

The invention also provides an agricultural composition comprising a compound of formula I in admixture with an agronomically acceptable diluent or carrier.

The composition of the invention may of course include

more than one compound of the invention.

In addition the composition can comprise one or more additional active ingredients, for example compounds known to possess plant-growth regulant, herbicidal, fungicidal, insecticidal or acaricidal properties. Alternatively the compounds of the invention can be used in sequence with the other active ingredient. Fungicides which can be used in conjunction with the compounds of the present invention include maneb, zineb, mancozeb, thiram, ditalimfos, tridemorph, fenpropimorph, imazalil, propiconazole, flutriafol, penconazole, triadimefon, triadimenol, diclobutrazol, fluotrimazole, ethirimol, fenarimol, nuarimol, triforine, pyracarbolid, tolclofos-methyl, oxycarboxin, carbendazim, benomyl, thiophanate, thiophanate-methyl, thiabendazole, propineb, metalaxyl, dicloran, dithianon, fuberidazole, dodine, chlorothalonil, cyprofuram, dichlofluanid, sulphur, copper compounds, iprodione, ziram, nabam, prochloraz (and metal complexes of this e.g. the manganese chloride complex), zineb-ethylene thiuramsulphide adduct, captan, captafol, benodanil, mepronil, carboxin, guazatine, validamycin, vinclozolin, tricyclazole, quintozene, pyrazophos, furmecyclox, propamocarb, prothiocarb, procymidone, kasugamycin, furalaxyl, folpet, fenfuram, ofurace, oxadixyl, etridiazole, fosetyl aluminium, methfuroxam,

fenapanil, fentin hydroxide, IBP, cycloheximide, binapacryl, dodemorph, dimethirimol, bupirimate, nitrothal-isopropyl, quinomethionate, bitertanol, flutolanil, etaconazole, fenpropidine, flubenzimine, cymoxanil and benalaxyl. Plant-growth regulants with which the products can be mixed include chlormequat, mepiquat, ethephon, paclobutrazol, dikegulac-sodium, gibberellic acid, ancymidol, maleic hydrazide, mefluidide and daminozide.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated

naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols.

Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amide prepared by the condensation of a carboxylic acid with a di- or polyamine; or a quaternary ammonium salt.

The compositions of the invention can take any form known in the art for the formulation of fungicidal, plant growth regulant and similar compounds, for example, a

solution, a dispersion, an aqueous emulsion, a dusting powder, a seed dressing, a fumigant, a smoke, a dispersible powder, an emulsifiable concentrate or granules. Moreover it can be in a suitable form for direct application or as a concentrate or primary composition which requires dilution with a suitable quantity of water or other diluent before application.

As a dispersion, the composition comprises a compound of the invention dispersed in a liquid medium, preferably water. It is often convenient to supply the consumer with a primary composition which can be diluted with water to form a dispersion having the desired concentration. The primary composition can be provided in any one of the following forms. It can be a dispersible solution which comprises a compound of the invention dissolved in a water-miscible solvent with the addition of a dispersing agent. A further alternative comprises a compound of the invention in the form of a finely ground powder in association with a dispersing agent and intimately mixed with water to give a paste or cream which can if desired be added to an emulsion of oil in water to give a dispersion of active ingredient in an aqueous oil emulsion.

An emulsifiable concentrate comprises a compound of the invention dissolved in a water-immiscible solvent which is formed into an emulsion with water in the

presence of an emulsifying agent.

A dusting powder comprises a compound of the invention intimately mixed and ground with a solid pulverulent diluent, for example, kaolin.

A granular solid comprises a compound of the invention associated with similar diluents to those which may be employed in dusting powders, but the mixture is granulated by known methods. Alternatively it comprises the active ingredient adsorbed or absorbed on a pre-granular diluent, for example, Fuller's earth, attapulgite or limestone grit.

A wettable powder usually comprises the active ingredient in admixture with a suitable surfactant and an inert powder diluent such as china clay.

Another suitable concentrate is a flowable suspension concentrate which is formed by grinding the compound with water, a wetting agent and a suspending agent. The concentration of the active ingredient in the composition of the present invention, as applied to plants is preferably within the range of 0.01 to 3.0 per cent by weight, especially 0.01 to 1.0 per cent by weight. In a primary composition the amount of active ingredient can vary widely and can be, for example, from 5 to 95 per cent by weight of the composition.

In the method of the invention the compound is generally applied to seeds, plants or their habitat.

Thus, the compound can be applied directly to the soil before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid form of granules, or by applying the active ingredient at the same time as drilling by inserting it in the same drill as the seeds. A suitable applications rate is within the range of from 0.05 to 20 kg per hectare, more preferably from 0.1 to 10 kg per hectare.

Alternatively the active compound can be applied directly to the plant by, for example, spraying or dusting either at the time when the fungus has begun to appear on the plant or before the appearance of fungus as as protective measure. In both such cases the preferred mode of application is by foliar spraying. It is generally important to obtain good control of fungi in the early stages of plant growth as this is the time when the plant can be most severely damaged. For cereal crops such as wheat, barley and oats it is often desirable to spray the plant at or before growth stage 5 although additional treatments by spraying when the plant is more mature can augment resistance to the growth or spread of fungi. The

spray or dust can conveniently contain a pre- or post-emergence herbicide if this is thought necessary. Sometimes, it is practicable to treat the roots of a plant before or during planting, for example, by dipping the roots in a suitable liquid or solid composition. When the active compound is applied directly to the plant a suitable rate of application is from 0.01 to 10 kg. per hectare, preferably from 0.05 to 5 kg per hectare.

When the compounds of the invention are used as growth regulators they are usually best applied during the vegetative stages of growth at similar rates as above. In the case of crops such as legumes, cotton and sunflowers, application preferably takes place during the late vegetative stage of growth, or just prior to or just after the onset of flowering. In the case of cereals, pot plants and turf, earlier application, during the early vegetative stage of growth, is more appropriate.

The compounds of the invention may be prepared in variety of ways as shown by the following reaction sequences. (X = halogen)

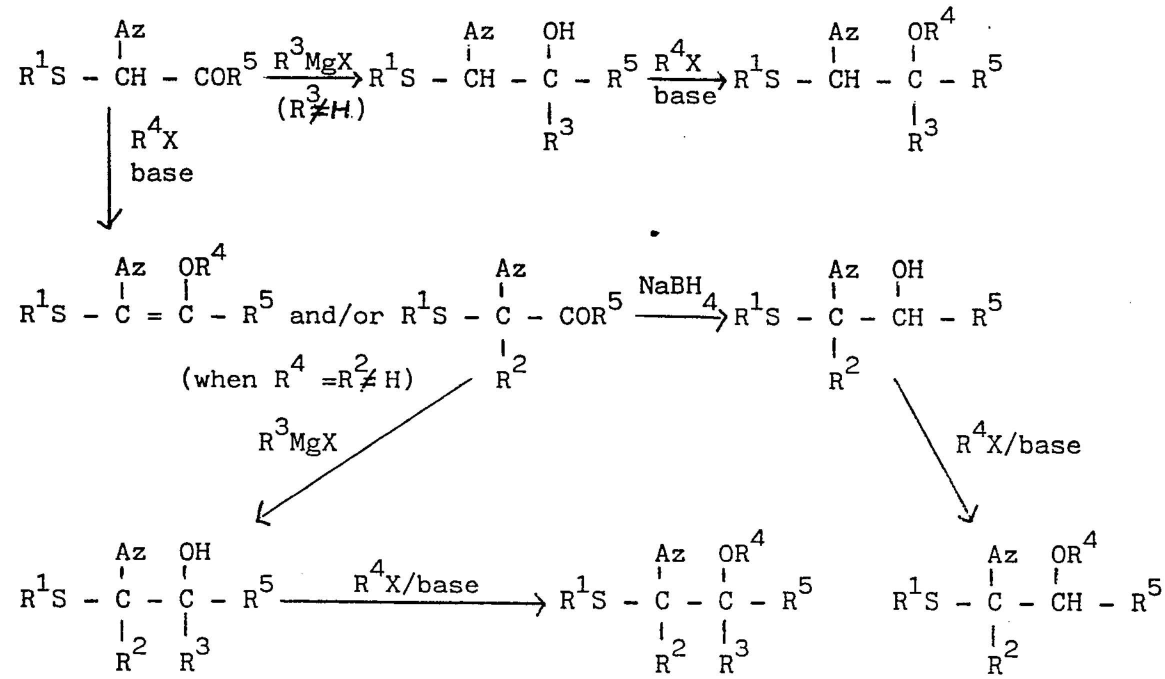


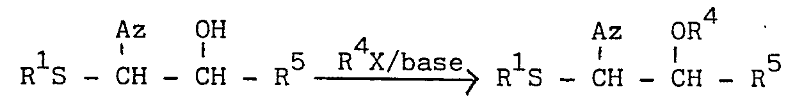


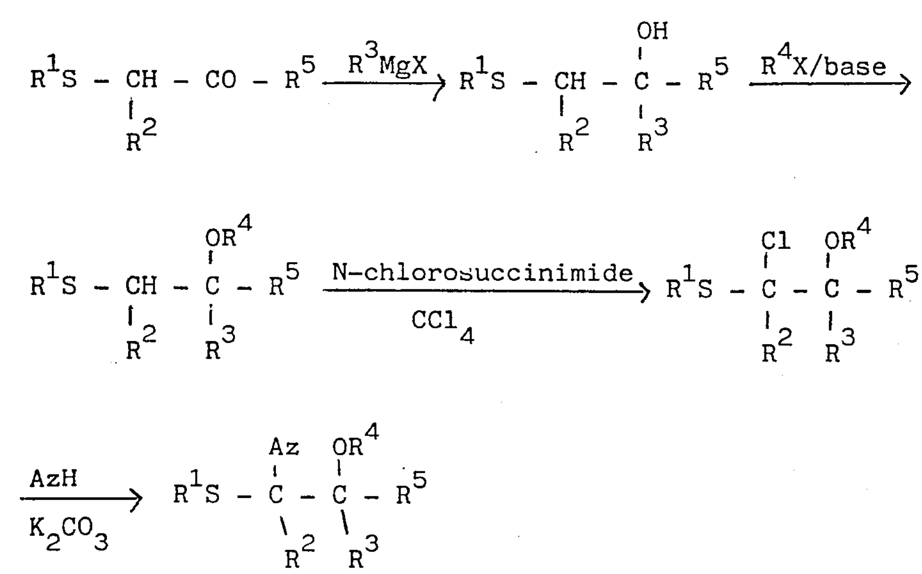

Compounds in which n is 1 may be obtained by oxidising compounds of formula I in which n is 0. Compounds in which n is 2 may be obtained by oxidising compounds of formula I in which n is 0 or 1. As oxidising agent there may be used a per acid, e.g. 3-chloroperbenzoic acid. The molar amount of oxidising agent will usually determine the degree of oxidation.

Salts can be prepared by reacting the free base with a suitable acid e.g. hydrochloric acid. Complexes can be prepared by reacting the base with a suitable metal salt e.g. manganese (II) chloride.

The invention is illustrated in the following Examples. Structures of isolated novel compounds were confirmed by elemental and/or other appropriate analyses.

Example 1

Sodium hydride (50% in oil, 1.1 g) was washed by decantation with petroleum ether and added as a slurry in dimethyl formamide (10 ml) to a stirred solution of 1-[1-t-butylthio-2-(4-chlorophenyl)-2-hydroxyethyl]-1H-1,2,4 -triazole (6.0 g) in dimethylformamide (40 ml) at about -20°C. The mixture was stirred for 5 minutes at -10 to -20°C and then a solution of methyl iodide (3.2 g) in dimethyl formamide (10 ml) was added dropwise at -10 to -20°C. The temperature was maintained at about -10°C for 10 minutes then allowed to rise to room temperature and

stirring continued for 1½ hours. The mixture was added to water, extracted with ether and the ether extract, washed with water, dried and evaporated under reduced pressure. The residue was recrystallised from cyclohexane to give 1-[1-t-butylthio-2-(4-chlorophenyl)-2-methoxyethyl]-1H-1,2,4 -triazole, mp 99-101°C. (Compound 1).

<u>Example 2</u>

In a similar manner to Example 2 reaction of the starting material of Example 1 with acetyl chloride gave 1-[2-acetoxy-1-t-butylthio-2-(4-chlorophenyl)ethyl]-1H-1,2,4 - triazole, obtained as a colourless oil. (Compound 2).

<u>Example 3</u>

A solution of 1-[1-t-butylthio-2-(4-chlorophenyl) -2-oxoethyl]-1H-1,2,4-triazole (10.2 g) in tetrahydrofuran (30 ml) was added dropwise to methyl magnesium iodide prepared from methyl iodide (5.7 g) and magnesium (1.0 g) in dry ether (100 ml) with stirring at about 10°C. Stirring was continued at room temperature for 2 hours. Ice cold water was then added dropwise at 5-10°C followed by enough aqueous ammonium chloride to dissolve the magnesium residues. The organic phase was separated, washed with water (3 times), dried over magnesium sulphate and evaporated under reduced pressure giving crude product as a semi-solid. Trituration with petroleum ether (bp 80-100°C) gave a white solid which was heated in

di-isopropyl ether and filtered to give 1-[1-t-butylthio-2-(4-chlorophenyl)-2-hydroxypropyl]-1H-1,2,4-triazole, mp 152-3°C. (Compound 3).

Example 4

In a similar manner to Examples 1,2 or 3 using the appropriate starting materials the following were obtained. In the table, in the column headed Az I = 1-imidazolyl and T = 1-(1,2,4-triazolyl).

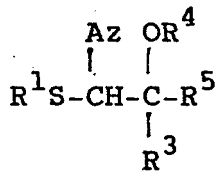

| Cpd No | Az | $R^1$ | $R^3$ | $R^4$ | $R^5$ | mp (°C) |
|---|---|---|---|---|---|---|
| 4 | T | $Bu^t$ | H | $-CH_2CH=CH_2$ | 4ClPh | 94-5 |
| 5 | T | $Bu^t$ | H | $Bu^n$ | 4ClPh | oil |
| 6 | T | $Bu^t$ | H | Me | $2,4Cl_2Ph$ | 101-2 |
| 7 | T | $Bu^t$ | H | $-CH_2COOEt$ | 4ClPh | oil |
| 8 | I | $Bu^t$ | H | Me | 4ClPh | oil |
| 9 | T | $EtC(Me_2)-$ | H | Me | 4ClPh | 91-3 |
| 10 | T | $Pr^i$ | H | Me | 4ClPh | 75-8 |

| Cpd No | Az | $R^1$ | $R^3$ | $R^4$ | $R^5$ | mp (°C) |
|---|---|---|---|---|---|---|
| 11 | T | $Bu^t$ | H | Et | 4ClPh | 109-12 |
| 12 | T | $EtC(Me_2)-$ | H | Et | 4ClPh | 83-5 |
| 13 | T | $EtC(Me_2)-$ | H | $Bu^t$ | 4ClPh | 63-6 |
| 14 | T | $Bu^t$ | H | $-CH_2-CH=CH_2$ | 4ClPh | oil |
| 15 | T | $Bu^t$ | H | $PhO(CH_2)_2$ | 4ClPh | 100-2 |
| 16 | T | $EtC(Me_2)-$ | H | $4Cl-PhCH_2$ | 4ClPh | oil |
| 17 | T | $Bu^t$ | H | $ClCH_2CO-$ | 4ClPh | oil |
| 18 | T | $Bu^t$ | H | PhNHCO- | 4ClPh | 170-5 |
| 19 | T | $EtC(Me_2)-$ | H | Me | $2,4Cl_2Ph$ | 57-9 |
| 20 | T | $Pr^i$ | H | Me | $2,4Cl_2Ph$ | 75-7 |
| 21 | T | $Bu^t$ | H | Et | $2,4Cl_2Ph$ | 45-7 |
| 22 | T | $Bu^t$ | H | $CH_2=CHCH_2-$ | $2,4Cl_2Ph$ | 96-8 |
| 23 | T | $Bu^t$ | H | $CH_2=C(Me)CH_2-$ | $2,4Cl_2Ph$ | 97-9 |
| 24 | T | $Bu^t$ | H | Me | 4BrPh | 97-9 |
| 25 | T | $Bu^t$ | H | $CH_2=CHCH_2-$ | 4BrPh | 104-6 |
| 26 | T | $Bu^t$ | Me | Me | 4ClPh | 139-40 |
| 27 | T | $Bu^t$ | Et | Me | 4ClPh | oil |
| 28 | I | $Bu^t$ | Me | $CH_2=CHCH_2-$ | 4ClPh | 153-9 |

| Cpd No | Az | $R^1$ | $R^3$ | $R^4$ | $R^5$ | mp (°C) |
|---|---|---|---|---|---|---|
| 29 | T | $Bu^t$ | Me | H | $2,4Cl_2Ph$ | 176-80 |
| 30 | I | 4ClPh | Me | Me | Me | 78-80 |
| 31 | T | 4ClPh | Me | Me | Me | 58-60 |
| 32 | T | 4ClPh | H | Me | $Bu^t$ | 79-108 |
| 33 | T | $Bu^t$ | H | Me | $4CF_2HOPh$ | oil |
| 34 | T | $EtC(Me_2)-$ | H | Me | $4CF_2HOPh$ | oil |
| 35 | T | $EtC(Me_2)-$ | H | Et | $4CF_2HOPh$ | oil |

Example 5

Sodium methoxide (2.9 g) was added to 1-[t-butylthio-2-(4-chlorophenyl)-2-oxoethyl]-1H-1,2,4-triazole (15 g) in dimethylformamide (70 ml) with stirring and cooling followed by ethyl iodide (8.3 g). The mixture was heated slowly to 80°C, maintained at this temperature for 1 hour, heated to 110°C and maintained at this temperature for 2 hours. Addition to water and isolation through ether gave crude product, shown by NMR to be a mixture of (E) and (Z) isomers in the ratio 2:1. Chromatography on silica gel using 3:1 ether/petroleum ether (bp 60-80°C) gave two

fractions; The first was recrystallised from petroleum ether (bp 60-80°C) to give (Z)-1-[1-t-butyl-2-(4-chlorophenyl)-2-ethoxyethenyl]-1H-1,2,4-triazole, mp 65-7°C. (Compound 36).

The second fraction was dissolved in ether and treated with a solution of hydrogen chloride in diisopropyl ether (10 ml of 14.5% solution). The precipitated hydrochloride was washed with 1:1 acetone/ether and then basified by stirring with aqueous sodium bicarbonate and ether. Separation of the ether solution and work-up followed by recrystallistation from petroleum ether (bp 80-100°C) gave the (E)-isomer, mp 73-5°C. (Compound 37).

In a similar manner the following products were obtained. In some cases only one isomer (usually the E-isomer) was isolated when $R^4$ is a carbonyl derivative, the starting material was reacted with the appropriate carbonyl chloride in the presence of triethylamine.

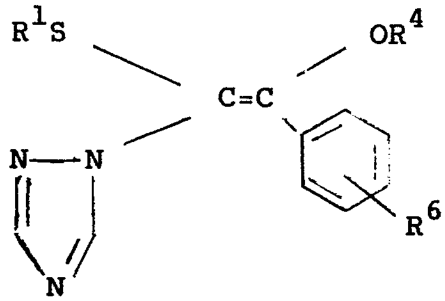

| Cpd E | Cpd Z | $R^1$ | $R^4$ | $R^6$ | mp (°C) E | mp (°C) Z |
|---|---|---|---|---|---|---|
| 38 | 39 | $Bu^t$ | Me | 4Cl | 80-1 | 112-3 |
| 40 | 41 | $Bu^t$ | $Bu^i$ | 4Cl | 72-4 | oil |
| 42 | | $EtC(Me_2)-$ | Et | 4Cl | oil | |
| | 43 | $Pr^i$ | Me | 4Cl | | 71-4 |
| 44 | 45 | $Bu^s$ | Me | 4Cl | oil | 44-5 |
| 46 | 47 | $Bu^t$ | $Pr^n$ | 4Cl | 75-7 | oil |
| 48 | 49 | $Pr^i$ | $Pr^n$ | 4Cl | 58-9 | oil |
| 50 | 51 | $Bu^t$ | $4ClPhCH_2$ | 4Cl | 103-4 | 100-2 |
| 52 | | $Bu^t$ | Me | $2,4Cl_2$ | 69-72 | |
| 53 | 54 | $EtC(Me_2)-$ | Me | $2,4Cl_2$ | oil | 75-7 |
| 55 | | $Bu^t$ | Et | $2,4Cl_2$ | 94-6 | |
| 56 | | $EtC(Me_2)-$ | Et | $2,4Cl_2$ | oil | |
| 57 | 58 | $Pr^n$ | Et | $2,4Cl_2$ | oil | 42-6 |

| Cpd E | Cpd Z | $R^1$ | $R^4$ | $R^6$ | mp (°C) E | mp (°C) Z |
|---|---|---|---|---|---|---|
| 59 | 60 | $Bu^t$ | $CH_2=CHCH_2$ | $2,4Cl_2$ | 85-7 | oil |
| 61 | 62 | $Bu^t$ | $MeCOO(CH_2)_2$ | 4Cl | oil | oil |
| 63 | | $Bu^t$ | $Pr^n$ | $2,4Cl_2$ | 84-6 | |
| 64 | 65 | $EtC(Me_2)-$ | $Pr^n$ | $2,4Cl_2$ | oil | oil |
| 66 | 67 | $Bu^t$ | $Bu^n$ | $2,4Cl_2$ | oil | oil |
| 68 | 69 | $Bu^t$ | $PhCH_2$ | $2,4Cl_2$ | 102-4 | oil |
| 70 | 71 | $EtC(Me_2)-$ | $4ClPhCH_2$ | $2,4Cl_2$ | 106-8 | oil |
| 72 | | $Bu^t$ | $HO(CH_2)_2$ | $2,4Cl_2$ | 117-9 | |
| 73 | | $Bu^t$ | Et | 4Br | 85-6 | |
| 74 | 75 | $Bu^t$ | MeCO | 4Cl | 73-5 | 150-2 |
| 76 | 77 | $Bu^t$ | EtOCO | 4Cl | 107-8 | 98-9 |
| 78 | 79 | $Bu^t$ | Me | $4CF_2HO$ | oil | oil |
| 80 | 81 | $Bu^t$ | Et | $4CF_2HO$ | oil | oil |
| 82 | 83 | $Bu^t$ | Et | $3CF_3$ | 55-7 | 46-52 |
| 84 | 85 | | Me | 4Cl | 77-80 | oil |

| Cpd E | Cpd Z | $R^1$ | $R^4$ | $R^6$ | mp (°C) E | mp (°C) Z |
|---|---|---|---|---|---|---|
| 86 | 87 | $Bu^t$ | $2ClPhCH_2$ | 4Cl | oil | 61-3 |
| 88 | 89 | $Bu^t$ | $Pr^i$ | 4Cl | 91-3 | 99-101 |
| 90 | 91 | $Bu^t$ | 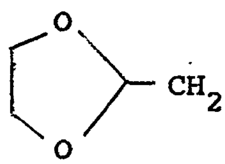 | 4Cl | 132-3 | 83-4 |

Example 6

During the reactions of Example 5 a C-alkylated product is usually obtained. In some cases this is isolated as a fraction of the chromatography separation. In this way the following compounds were obtained.

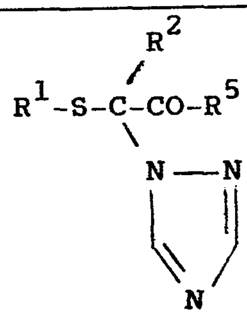

| Cpd. No | $R^1$ | $R^2$ | $R^5$ | mp (°C) |
|---|---|---|---|---|
| 92 | $Bu^s$ | Me | 4ClPh | oil |
| 93 | $Bu^t$ | Me | 4ClPh | 101-2 |
| 94 | $Pr^i$ | Me | 4ClPh | oil |
| 95 | 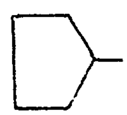 | Me | 4ClPh | 72-4 |
| 96 | $Bu^t$ | Me | $4CF_2HOPh$ | 72-3 |
| 97 | $Bu^t$ | Et | $4CF_2HOPh$ | oil |
| 98 | 4Cl-Ph | Me | $Bu^t$ | 132-3 |

Example 7

Compound 98 was reduced by treatment with sodium borohydride followed by hydrochloric acid and then conventional work-up to give 1-[1-(4-chlorophenylthio)-2-hydroxy-1,3,3-trimethylbutyl]-1H-1,2,4-triazole, mp. 102-12. (Compound 99).

Example 8

m-Chloroperbenzoic acid (2.5 g of 85% pure material) was added portionwise to compound 86 (2.4 g) in dichloromethane (40 ml) with stirring and cooling. The mixture was stirred for six hours at room temperature and left for 2 days. The mixture was treated with aqueous sodium bicarbonate and the organic layer washed with water, dried and evaporated. The residue was dissolved in either, filtered and the filtrate evaporated. The residue was recrystallised from ethanol to give (E)-1-[1-t-butylsulphonyl-2-(4-chlorophenyl)-2-(2-chlorobenzyloxy)ethenyl]-1H-1,2,4-triazole, m.p. 145-7°C (Compound 100).

Example 9

By treatment with the appropriate acid in organic solvent the following salts were obtained.

| Salt No | Acid | Compound No | m.p.(°C) |
|---|---|---|---|
| 1 | Oxalic | 14 | 176-197 |
| 2 | Hydrochloric | 19 | 170-5 |
| 3 | Hydrochloric | 42 | 154-8 |
| 4 | Hydrochloric | 73 | 147-52 |

Example 10

The compounds of the invention were subjected to various tests.

a) Fungicide tests

Compounds are assessed for activity against one or more of the following:

Erysiphe graminis: barley powdery mildew (EG).

Phytophthora infestans: potato blight (PI)

Plasmopara viticola: vine downy mildew (PV)

Puccinia recondita: brown leaf rust of wheat (PR)

Pyricularia oryzae: rice blast (PO)

The compounds are formulated in aqueous acetone with Tween 20 wetter to give a concentration of 500 ppm compound/125 ppm wetter/20,000 ppm acetone. For cereals, Pluronic L61 (ethylene oxide/propylene oxide block copolymer) is added (1000 ppm) as an additional wetter. Plants are then treated with the diluted suspensions and then inoculated, 24 hours after treatment with test compound, by spraying with spore suspensions of the fungi and then incubating in a humid atmosphere: >98% RH, or by shaking diseased material over the treated plants for the EG, as summarised in Table 1.

Table 1 Environmental conditions during incubation

| Pathogen | Incubation time (days) | Temperature day | Temperature night | Light conditions | Duration of high humidity (days) |
|---|---|---|---|---|---|
| *P. infestans* | 4 | 14 | 10 | 17 hrs light/ 7 hrs dark per day | 1 |
| *P. viticola* | 11 | 18 | 14 | 16 hrs light/ 8 hrs dark per day | 11 |
| *P. recondita* | 12 | 18 | 14 | 1 day dark, 16 hrs light/ 8 hrs dark per day | 1 |

| Pathogen | Incubation time (days) | Temperature day | Temperature night | Light conditions | Duration of high humidity (days) |
| --- | --- | --- | --- | --- | --- |
| <u>P. oryzae</u> | 7 | 24 | 18 | 3 days dark then 14 hrs light/10 hrs dark per day | 7 |
| <u>E. Graminis</u> | 9 | 18 | 14 | As for PV | |

After the appropriate period of incubation, the degree of infection of the leaf surface is visually estimated.

Compounds are considered active if they give greater then 50% control of the disease at a concentration of 2000 ppm (w/v) or less.

b) <u>Plant growth regulant tests (PGR)</u>

Mung bean (MB) seeds were sown in pots containing coarse grade vermiculite (3-5 seeds per 6cm pot). Five days

later each pot was placed in approximately 100 ml of an aqueous dispersion of the chemical under test and shoots which had emerged were sprayed to run-off with a portion of test liquid. Eight days later the heights of the seedlings were measured and compared with control plants. Similar tests were also carried out on barley (B) and sunflower (S). Compounds are consided active if they gave a reduction of at least 20% in height compared with controls at rate of 100 mg/L or less.

Activities were demonstrated as follows (+=active).

| Compound No | FUNGICIDE EG | PI | PO | PR | PV | PGR MB | B | S |
|---|---|---|---|---|---|---|---|---|
| 19 | + | | + | | | + | | |
| 20 | + | | | | | | | |
| 21 | + | | | | | | | |
| 22 | + | | + | | | + | | |
| 23 | + | | | | | + | | + |
| 24 | + | | + | | | + | + | + |
| 25 | + | | + | + | | | + | |
| 26 | | | | | | + | + | + |
| 27 | | | | | | + | | + |
| 28 | + | | + | | | | | |
| 29 | + | | | + | | | + | |
| 30 | + | | | + | | + | | |
| 31 | + | | | | | | | + |
| 32 | + | | + | | | + | + | |
| 33 | | | | | + | | | |
| 35 | | | | | + | | | |
| 36 | + | | | | | + | + | |
| 38 | + | | | | | + | | + |

| Compound No | FUNGICIDE EG | PI | PO | PR | PV | PGR MB | B | S |
|---|---|---|---|---|---|---|---|---|
| 39 | + | | | | | + | | |
| 40 | + | | | | | | | |
| 41 | + | | | | | + | | |
| 42 | + | | + | + | | + | + | + |
| 43 | + | | | | | | | |
| 44 | + | | | | | | | |
| 45 | + | | | | | | | |
| 46 | + | | + | | | | | |
| 47 | + | | + | | | | + | |
| 48 | + | | + | | | + | + | + |
| 49 | + | | + | | | + | + | + |
| 50 | + | | + | | | | | + |
| 51 | + | | + | | | | | |
| 52 | + | | + | | | + | | |
| 53 | | | | | | + | | |
| 55 | + | | + | | | + | | + |
| 56 | + | | | | | | | |
| 57 | | | | | | + | | |

| Compound No | FUNGICIDE EG | PI | PO | PR | PV | PGR MB | B | S |
|---|---|---|---|---|---|---|---|---|
| 59 | + | | | | + | | + | |
| 60 | + | | | | | | + | |
| 61 | + | | + | | | | | |
| 62 | | | | | | | | + |
| 63 | + | | + | | | + | | |
| 68 | + | | | | | + | + | + |
| 69 | + | | | | | | + | |
| 72 | | | | | | + | | |
| 73 | + | | + | + | | + | | |
| 74 | + | | | + | | | | |
| 75 | + | | | + | | | + | |
| 76 | + | | | | + | | | |
| 77 | + | | | | + | | | |
| 78 | + | | | | | | | |
| 80 | + | | + | | + | | | |
| 81 | | | | | + | + | | |
| 83 | + | | | | + | + | | |
| 93 | + | | | | | | | |

| Compound No | FUNGICIDE EG | PI | PO | PR | PV | PGR MB | B | S |
|---|---|---|---|---|---|---|---|---|
| 94 | | | | | | + | | + |
| 98 | + | | | | | | + | + |
| Salt 1 | + | | | | | | + | + |
| 2 | + | | + | | | | | |
| 3 | + | | + | + | | | | |
| 4 | + | | + | + | | + | | |

CLAIMS

1. A compound of formula I.

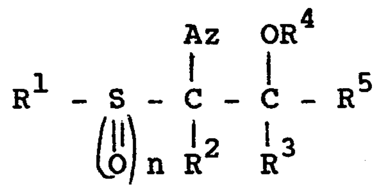

I

where i) Az is 1-imidazolyl or 1-(1,2,4-triazolyl);

ii) $R^1$ and $R^5$ are aryl, cycloalkyl or optionally substituted alkyl;

iii) $R^4$ is hydrogen, alkenyl, cycloalkyl, optionally substituted alkyl, aryl, optionally substituted alkanoyl, optionally substituted benzoyl, alkoxycarbonyl or optionally substituted carbamoyl;

iv) n is 0,1 or 2;

v) (a) $R^2$ is hydrogen, optionally substituted alkyl, cycloalkyl or alkenyl and $R^3$ is hydrogen or optionally substituted alkyl, or

(b) $R^2$ and $R^3$ form a double bond;

vi) when $R^2$ is optionally substituted alkyl, cycloalkyl or alkenyl and Az is 1-(1,2,4-triazolyl), $R^3$ and $R^4$ can also

form a double bond;

together with acid addition salts, complexes of these compounds with metal salts, with the provisos

a) at least one of $R^2$, $R^3$ and $R^4$ is not hydrogen,

b) when $R^1$ is aryl and $R^2$ and $R^3$ are hydrogen, $R^4$ is not alkoxymethyl,

c) when $R^1$ is aryl or aralkyl, $R^2$ and $R^3$ are hydrogen and $R^5$ is unsubstituted alkyl, $R^4$ is not alkanoyl, and

d) when $R^2$ is substituted benzyl and $R^3$ and $R^4$ form a double bond, $R^5$ is not tertiary butyl.

2. A compound according to Claim 1 in which n is O.

3. A compound according to Claim 1 or 2 in which $R^1$ is $C_{4-5}$ alkyl or halophenyl.

4. A compound according to Claim 1,2 or 3 in which $R^2$ and $R^3$ are hydrogen or methyl or form a double bond.

5. A compound according to any one of the preceding claims in which $R^5$ is $C_{1-4}$ alkyl or halophenyl.

6. A compound according to any one of the preceding claims in which $R^4$ is $C_{1-4}$ alkyl.

7. A compound according to any one of Claims 1 to 5 in

which $R^3$ is methyl and $R^4$ is hydrogen.

8. A compound according to any one of the preceding claims in which Az is 1-(1,2,4-triazolyl).

9. An agricultural composition comprising a compound claimed in any of the preceding in admixture with an agronomically acceptable diluent or carrier.

RDW/KJT

I.D. 0495I

17th September 1984

European Patent Office

# EUROPEAN SEARCH REPORT

0140571

Application number

EP 84306453.6

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| D,A | EP - B1 - 0 000 752 (BASF)<br>* Claims 1,2 *<br>-- | 1,9 | C 07 D 249/08<br>C 07 D 233/60<br>A 01 N 43/653<br>A 01 N 43/50 |
| A | DE - B2 - 2 814 355 (BASF)<br>* Claim 1 *<br>-- | 1 | |
| A | EP - A3 - 0 065 204 (BAYER)<br>* Abstract *<br>-- | 1,9 | |
| A | DE - A1 - 3 200 414 (BAYER)<br>* Formula I; claim 4 *<br>-- | 1,9 | |
| A | CHEMICAL ABSTRACTS, vol. 99, no. 3, July 18, 1983, Columbus, Ohio, USA<br>IMPERIAL CHEMICAL INDUSTRIES PLC "Triazole and imidazole compounds as fungicides and plant growth regulators"<br>page 622, column 2, abstract-no. 22 478h<br>& Jpn. Kokai Tokkyo Koho JP 58 39, 671 [83 39,671]<br>---- | 1 | TECHNICAL FIELDS SEARCHED (Int. Cl.4)<br>C 07 D 249/00<br>C 07 D 233/00 |

The present search report has been drawn up for all claims

| Place of search | Date of completion of the search | Examiner |
|---|---|---|
| VIENNA | 07-12-1984 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82